# EUROPEAN PATENT APPLICATION

(11) **EP 4 403 179 A1**
(43) Date of publication of application: **24.07.2024**
(21) Application number: 22867757.1
(22) Date of filing: 13.09.2022
(51) Int. Cl.: A61K 35/17, A61K 9/00, A61P 25/28

(54) **PHARMACEUTICAL COMPOSITION CONTAINING REGULATORY T CELLS AS ACTIVE INGREDIENT FOR PREVENTING OR TREATING DEGENERATIVE BRAIN DISEASES**

(30) Priority: 13.09.2021 KR 20210121783
(71) Applicant: VT Bio Co., Ltd., Gwangju 62465 (KR)
(72) Inventor: LEE, Seungwon, Seoul 05070 (KR); KIM, Jae Yoon, Namyangju-si Gyeonggi-do 12207 (KR); KIM, Hyung Joon, Seoul 05667 (KR); PARK, Hyeonwoong, Seoul 052401 (KR)
(74) Representative: Nederlandsch Octrooibureau
(86) International application number: PCT/KR2022/013610
(87) International publication number: WO 2023/038489

(57) **Abstract**

The present invention relates to a pharmaceutical composition for preventing or treating degenerative brain disease, containing regulatory T cells as an active ingredient. The pharmaceutical composition according to the present invention inhibits M1 microglial activation or transformation into DAM, promotes M2 polarization, inhibits tau protein phosphorylation, and increases the expression of anti-inflammatory cytokines, thereby protecting nerve cells, and thus it may be useful for the prevention or treatment of degenerative brain diseases.

## Description

### Technical Field

The present invention relates to a pharmaceutical composition for preventing or treating degenerative brain disease comprising regulatory T cells as an active ingredient.

### Background Art

Alzheimer's disease (AD) is a common neurodegenerative disease characterized by progressive loss of memory and cognitive functions. To date, the cause of Alzheimer's disease has not been fully elucidated, and neuroinflammation is believed to be a central process in the pathogenesis of Alzheimer's disease. Studies on Alzheimer's disease patients provide evidence for persistent neuroinflammation in the early stages of Alzheimer's disease preceding neuronal atrophy.

Meanwhile, recent advances in the field of immunotherapy have generated new powerful approaches for targeting for neurological diseases. Specifically, regulatory T cell therapy is reshaping the landscape of brain-related disorder treatment. Regulatory T cells (Tregs) are a small subset of T cells that modulate the immune system and maintain immune homeostasis. CD4+CD25+Foxp3+ regulatory T cells account for 5 to 10% of CD4+ T cells and play an important role in inducing immune tolerance and maintaining immune homeostasis. Studies on patients and animal models of ischemic stroke have demonstrated the therapeutic efficacy of Treg cells that block hemorrhage and improve sensorimotor functions. In addition, when regulatory T cells were administered intravenously to ischemic mice, hemorrhagic transformation in the brain was dramatically alleviated, suggesting a neuroprotective effect mediated by the infused Treg cells. Treg cells have been studied for use in the treatment of neurodegenerative diseases in addition to autoimmune brain diseases, but it still remains unclear whether injection of Treg cells is helpful in treating Alzheimer's disease.

Microglia, the main immune cells of the central nervous system, play an important role in controlling brain inflammation. In Alzheimer's disease, activated microglia are known to modulate neuroinflammation by inducing various inflammatory responses. Microglia generally have two different phenotypes of activation: pro-inflammatory M1 and immunosuppressive M2. Microglia of the M1 phenotype mainly induce inflammatory responses by releasing inflammatory cytokines and chemokines and expressing genes essential for inflammatory responses. On the other hand, microglia of the M2 phenotype release many anti-inflammatory cytokines, chemokines, and anti-neurogenic factors to protect against or treat inflammatory responses. Therefore, inhibition of M1 polarization of microglia and induction of M2 polarization are expected to have a high potential therapeutic effect for Alzheimer's disease. Recent studies demonstrated that a disease-associated microglia (DAM) or "neurodegenerative" phenotype, defined by a set of upregulated genes, was observed in neurodegenerative diseases, including Alzheimer's disease, ALS, frontotemporal dementia, and aging. However, it remains unclear whether the loss of homeostatic function in microglia or the DAM phenotype is correlated with the degree of neuronal cell loss, and whether DAM is beneficial or detrimental to neurodegenerative diseases.

Therefore, the present inventors have sought to investigate the effect of human regulatory T cells (hTregs) injected intrathecally for the treatment of Alzheimer's disease, and have found that hTregs exhibit a neuroprotective effect by suppressing neuroinflammation in the brain and inhibiting the expression of inflammatory microglia, thereby completing the present invention.

### DISCLOSURE

### Technical Problem

An object of the present invention is to provide a pharmaceutical composition for preventing or treating degenerative brain disease comprising regulatory T cells as an active ingredient.

Another object of the present invention is to provide a method for preventing or treating degenerative brain disease.

### Technical Solution

To achieve the above objects, the present invention provides a pharmaceutical composition for preventing or treating degenerative brain disease comprising regulatory T cells as an active ingredient.

The present invention also provides a method for preventing or treating degenerative brain disease comprising a step of administering the pharmaceutical composition to a subject.

### Advantageous Effects

The pharmaceutical composition of the present invention protects nerve cells by inhibiting M1 microglial activation and transformation into DAM, promoting M2 polarization, inhibiting phosphorylation of tau protein, and increasing the expression of anti-inflammatory cytokines, thereby protecting nerve cells, and thus it may be useful for the prevention or treatment of degenerative brain disease.

### Brief Description of Drawings

FIG. 1 is a schematic diagram showing a process of preparing an animal model.
FIG. 2 depicts graphs showing the results of a passive avoidance test for AD mice.
FIG. 3 depicts graphs showing the mRNA expression levels of M1 markers in the hippocampus.
FIG. 4 depicts graphs showing the mRNA expression levels of DAM markers in the hippocampus.
FIG. 5 depicts graphs showing the protein expression levels of M1, M2, brain-derived nerve growth factor, and DAM markers in the hippocampus.
FIG. 6 depicts graphs showing the mRNA expression levels of M2 markers in the hippocampus.
FIG. 7 depicts photographs showing the results of immunofluorescence staining for CD116 and Trem2 in the hippocampus.
FIG. 8 photographs showing the results of immunofluorescence staining for phosphorylated tau protein in the hippocampus.

### Best Mode

Hereinafter, the present invention will be described in detail by way of embodiments with reference to the attached drawings. However, the following embodiments are provided to illustrate the present invention, and detailed description of a technology or configuration well known to those skilled in the art may unnecessarily obscure the subject matter of the present invention, the detailed description may be omitted. Also, the present invention is not limited by these embodiments. The present invention may be variously modified and applied within the scope of the appended claims and the equivalents interpreted therefrom.

In addition, the terminologies used in the present specification are term used to appropriately express preferred embodiments of the present invention, and these terms may change depending on the intention of a user or an operator, or customary practice in the art to which the present invention pertains. Accordingly, the definition of these terms should be made based on the contents throughout the present specification. Throughout the present specification, it is to be understood that when any part is referred to as "comprising" any component, it does not exclude other components, but may further comprise other components, unless otherwise specified.

Throughout the present specification, "%" used to indicate the concentration of a specific material, unless otherwise stated, refers to (w/w) % for solid/solid, (w/v) % for solid/liquid, and (v/v) % for liquid/liquid.

Hereinafter, the present invention will be described in more detail.

The present invention provides a pharmaceutical composition for preventing or treating degenerative brain disease comprising regulatory T cells as an active ingredient.

The term "regulatory T cells" as used in the present invention refers to a type of T cells that are uniquely capable of inducing immunosuppression, among T cell subtypes, and play a role in reducing excessively activated immune responses or suppressing unnecessary immune responses. The regulatory T cells control allergies, autoimmunity, inflammation, responses to microorganisms, etc. by suppressing immune responses.

In addition, the regulatory T cells may be human regulatory T cells, without being limited thereto.

In addition, the regulatory T cells are able to inhibit the expression of M1 microglia-related markers NOS2, CD86, IL-12a, IL-1β, IL-1β, IL-23, IL-6, and TNF-α.

In addition, the regulatory T cells are able to inhibit the expression of disease-associated microglia (DAM) markers Trem2, Tmem119, Cc3cr1, Itgax, and Ccl6.

In addition, the regulatory T cells are able to increase the expression of microglial M2 polarization-related markers IL-4, IL-10, TGF-β, Arg1, Ym1, Mrc1, and IL-2.

In addition, the degenerative brain disease may be selected from the group consisting of Alzheimer's disease, Parkinson's disease, dementia, progressive supranuclear palsy, multiple system atrophy, olivopontocerebellar atrophy (OPCA), Shy-Drager syndrome, striatonigral degeneration, Huntington's disease, amyotrophic lateral sclerosis (ALS), essential tremor, corticobasal ganglionic degeneration, diffuse Lewy body disease, Parkinson-ALS-dementia complex of Guam, and Pick's disease, but is not limited thereto and may be any neurological disease caused by neuroinflammation.

In addition, the pharmaceutical composition may be for intrathecal administration, without being limited thereto.

As used in the present invention, the term "prevention" used refers to any action that inhibits or delays the occurrence, development, and recurrence of degenerative brain disease by administration of the composition according to the present invention.

The term "treatment" as used in the present invention refers to any action that alleviates or beneficially changes the symptoms of degenerative brain disease and complications resulting therefrom by administration of the composition according to the present invention. Those of ordinary skill in the art to which the present invention pertains will know the exact criteria for the disease against which the composition of the present institution is effective, with reference to the data presented by the Korean Medical Association, etc., and determine the extent of amelioration, alleviation and treatment of the disease.

The composition according to the present invention may comprise a pharmaceutically effective amount of regulatory T cells alone or may further comprise one or more pharmaceutically acceptable carriers, excipients, or diluents. Here, the pharmaceutically effective amount refers to an amount sufficient to prevent, alleviate, and treat symptoms of immune disease. Here, "pharmaceutically acceptable" refers to an additive which is physiologically acceptable and, when administered to human beings, does not cause allergic reactions such as gastrointestinal disorders and dizziness, or similar reactions.

In addition, the composition of the present invention may be administered orally or parenterally in clinical practice and may be used in the form of a general pharmaceutical preparation. The mode of administration may be oral, mucosal (e.g., nasal, sublingual, vaginal, buccal, or rectal), parenteral (e.g., subcutaneous, intravenous, bolus injection, intramuscular, or intraarterial), topical (e.g., ocular), transdermal, or transcutaneous, without being limited thereto. Examples of dosage forms include, but are not limited to: tablets; caplets; capsules, such as soft elastic gelatin capsules; cachets; troches; lozenges; dispersions; suppositories; powders; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for administration by injection to a patient; eye drops or other ophthalmic preparations suitable for topical administration; and sterile solids (e.g., crystalline or amorphous solids) that can be reconstituted to provide liquid dosage forms suitable for administration by injection to a patient. The composition, shape, and type of dosage forms of the present invention will typically vary depending on their use. For example, a dosage form used in the acute treatment of a disease may comprise a larger amount of the active ingredient than a dosage form used in the chronic treatment of the same disease. Similarly, a parenteral dosage form may comprise a smaller amount of the active ingredient than an oral dosage form used to treat the same disease. The dosage forms and modes encompassed by the present invention are very diverse, which will be apparent to those skilled in the art to which the present invention pertains (see Remington's Pharmaceutical Sciences, 18th ed., Mack Publishing, Easton PA (1990))

In addition, the composition comprising a pharmaceutically acceptable carrier may be in various oral or parenteral dosage forms. The composition may be formulated using diluents or excipients such as fillers, extenders, binders, wetting agents, disintegrants, and surfactants, which are commonly used. The carriers, excipients or diluents may be one or more selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, saline, methyl hydroxybenzoate, propylhydroxy benzoate, talc, magnesium stearate, mineral oil, dextrin, calcium carbonate, propylene glycol, and liquid paraffin, without being limited thereto and any common carriers, excipients, or diluents may be used. The above-mentioned ingredients may be added independently or in combination to the regulatory T cells, which are the effective ingredient.

The pharmaceutical composition of the present invention may be administered orally or parenterally (e.g., intrathecally, intravenously, subcutaneously, intraperitoneally, or topically) according to the desired method, and the dosage thereof varies depending on the patient's body weight, age, gender, health status, and diet, the time of administration, the mode of administration, excretion rate, and the severity of the disease.

In addition, solid dosage forms for oral administration include tablets, powders, granules, capsules, etc., and such solid dosage forms may be prepared by mixing at least one compound with at least one excipient, such as starch, calcium carbonate, sucrose, lactose, gelatin, or the like. In addition to simple excipients, lubricants such as magnesium stearate, talc, etc. may also be used. Liquid dosage forms for oral administration include suspensions, oral liquids, emulsions, syrups, and the like, and may comprise, in addition to commonly used simple diluents such as water and liquid paraffin, various excipients such as wetting agents, sweeteners, fragrances, and preservatives.

In addition, dosage forms for parenteral administration include sterile aqueous solutions, non-aqueous solutions, suspensions, emulsions, freeze-dried preparations, suppositories, etc. As non-aqueous solvents and suspending agents, propylene glycol, polyethylene glycol, vegetable oil such as olive oil, injectable ester such as ethyl oleate, etc. may be used. As a base for suppositories, Witepsol, Macrogol, Tween 61, cacao butter, laurin butter, glycerol, gelatin, etc. may be used.

In addition, if necessary, the pharmaceutical composition of the present invention may be in the form of an injectable formulation including a sterile solution or suspension in water or another pharmaceutically acceptable liquid. For example, the pharmaceutical composition can be formulated by suitably combining the active ingredient with pharmaceutically acceptable vehicles or media, sterile water or saline, vegetable oil, an emulsifier, a suspension agent, a surfactant, a stabilizer, a flavoring compound, an excipient, a vehicle, a preservative, a binder, and the like, followed by mixing in a unit dose form required for generally accepted pharmaceutical practices. The amount of the active ingredient in the above formulation is such that a suitable dose within the designated range is provided.

Further, the sterile composition for injection can be formulated in accordance with conventional pharmaceutical practices using a vehicle such as distilled water for injection.

In addition, examples of an aqueous solution for injection include physiological saline or an isotonic solution containing glucose and other supplements such as D-sorbitol, D-mannose, D-mannitol, and sodium chloride, which may be used in combination with a suitable solubilizing agent, for example, alcohol such as ethanol or polyalcohol such as propylene glycol or polyethylene glycol, and a nonionic surfactant such as polysorbate 80^{™}, HCO-50, or the like.

In addition, examples of the oily liquid include sesame oil and soybean oil, which may be used in combination with benzyl benzoate or benzyl alcohol as a solubilizing agent. In addition, the oily liquid may be combined with a buffer such as a phosphate buffer or a sodium acetate buffer, a soothing agent such as procaine hydrochloride, a stabilizer such as benzyl alcohol or phenol, or an antioxidant. The prepared injectable solution is usually filled into an appropriate ampoule.

In addition, the pharmaceutical composition of the present invention is preferably administered parenterally to the patient's body. Specifically, the pharmaceutical composition is basically administered intrathecally once to three times, but may also be administered four times or more. In addition, the pharmaceutical composition may be administered for a short period of time or administered continuously for a long period of time. More specifically, the pharmaceutical composition may be an injectable dosage form or a transdermal dosage form. The injectable dosage form may be administered, for example, by intrathecal injection, intravenous injection, intraarterial injection, selective intraarterial injection, intramuscular injection, intraperitoneal injection, subcutaneous injection, intracerebroventricular injection, intracerebral injection, intramedullary injection, or the like.

In addition, the pharmaceutical composition of the present invention may be in any one dosage form selected from the group consisting of tablets, pills, powders, granules, capsules, suspensions, oral liquids, emulsions, syrups, sterile aqueous solutions, non-aqueous solutions, suspensions, emulsions, freeze-dried preparations, and suppositories. As a base for suppositories, Witepsol, Macrogol, Tween 61, cacao butter, laurin butter, glycerol, gelatin, etc. may be used.

In addition, the effective dosage of the active ingredient of the present invention for the human body may vary depending on the patient's age, weight, gender, and health status, dosage form, and the severity of the disease.

The pharmaceutical composition of the present invention may be used alone or in combination with surgery, hormone therapy, drug therapy, and methods that use biological response modifiers, to prevent or treat degenerative brain disease.

The present invention also provides a method for preventing or treating degenerative brain disease comprising a step of administering the pharmaceutical composition to a subject.

Since the method of the present invention includes the above-described pharmaceutical composition, the description of contents that overlap with those of the above-described pharmaceutical composition of the present invention will be omitted to avoid excessive complexity of the present specification due to the description of the overlapping contents.

The pharmaceutical composition of the present invention may be administered in a therapeutically effective amount or a pharmaceutically effective amount.

In the present invention, the term "therapeutically effective amount" refers to an amount of the pharmaceutically acceptable salt of the composition that is effective in preventing or treating the disease in the subject, and the therapeutically effective amount of the composition of the present invention may vary depending on several factors, for example, the mode of administration, the target area, and the patient's condition. Thus, the dosage when used in the human body should be determined in appropriate amounts in consideration of safety and efficacy. It is also possible to estimate the amount used in humans from the effective amount determined through animal experiments. These matters to be considered in determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

In the present invention, the term "pharmaceutically effective amount" refers to an amount that is sufficient to treat the disease at a reasonable benefit/risk ratio applicable to any medical treatment and that does not cause side effects. The effective dose level may be determined depending on factors, including the patient's health status, the kind and severity of the disease, the activity of the drug, sensitivity to the drug, the mode of administration, the time of administration, the route of administration, excretion rate, the duration of treatment, and drugs combined or used in combination with the composition, as well as other factors well known in the medical field. The composition of the present invention may be administered as an individual therapeutic agent or in combination with other therapeutic agents, and may be administered sequentially or simultaneously with conventional therapeutic agents. The composition may be administered in a single or multiple dosage form. It is important to administer the composition in the minimum amount that can exhibit the maximum effect without causing side effects, in view of all the above-described factors, and this amount can be easily determined by a person skilled in the art.

In addition, the pharmaceutical composition may be for intrathecal administration, without being limited thereto.

In the present invention, the "subject" is not particularly limited as long as it is a subject in need of the prevention or treatment of degenerative brain disease, and it preferably refers to mammals, including humans. The mammals include non-primates (cattle, pigs, horses, cats, dogs, rats, mice, etc.) and primates (humans, and monkeys such as cynomolgus monkeys and chimpanzees).

In addition, the degenerative brain disease may be selected from the group consisting of Alzheimer's disease, Parkinson's disease, dementia, progressive supranuclear palsy, multiple system atrophy, olivopontocerebellar atrophy (OPCA), Shy-Drager syndrome, striatonigral degeneration, Huntington's disease, amyotrophic lateral sclerosis (ALS), essential tremor, corticobasal ganglionic degeneration, diffuse Lewy body disease, Parkinson-ALS-dementia complex of Guam, and Pick's disease, but is not limited thereto and may be any neurological disease caused by neuroinflammation.

### Mode for Invention

Hereinafter, the present invention will be described in more detail by way of examples. These examples are for illustrating the present invention in more detail, and the scope of the present invention is not limited to these examples.

### <Example 1> Experimental Materials and Experimental Methods

### 1-1. Experimental Materials

Amyloid beta (Aβ₁₋₄₂) peptide was purchased from GenScript (Piscataway, NJ, USA). Aβ₁₋₄₂ peptide was suspended in 450 µL of distilled water (DW) and mixed with 50 µL of 10x phosphate buffered saline (PBS), thus preparing 1x PBS. To produce fibrillary Aβ, the dissolved peptide was incubated at 37°C overnight. bvPLA2 was purchased from Sigma (St. Louis, MO, USA), dissolved in PBS, and stored at -20°C until use.

### 1-2. Preparation of Animal Model

3xTg AD mice [B6;129-Tg(APPSwe,tauP301L)1Lfa Psen1^{tm1Mpm}/Mmjax] were purchased from Jackson Laboratory (Bar Harbor, ME, USA). A total of 18 male 3xTg AD mice were used. In the animal model, it was observed that extracellular Aβ occurred after 6 months of age, and tau pathology appeared after 12 months of age. All the mice were housed in a 12-hour light/dark cycle with ad libitum access to food and water, and 10-month-old mice were administered human regulatory T cells (hTregs). The mice were anesthetized with isoflurane, and a 25-gauge lumbar puncture needle was inserted into the spinal canal at the L3-L4 or L4-L5 levels. 1×10², 1×10³, 1×10⁴, or 1×10⁵ hTreg cells (contained in 10 µl of PBS) were infused into the cerebrospinal fluid over 20 seconds. Animal experiments were conducted in compliance with animal protection regulations, and the guidelines for animal experiments were approved by the Institutional Animal Care & Use Committee (IACUC) of Kyung Hee University (KHUASP(SE)-21-102).

### 1-3. Isolation of Mononuclear Cells

Mononuclear cells were isolated according to the following procedure.
1) Gently shake a blood pack to mix, and then pour the blood into a 500-mL square media bottle. Collect 30 µL of the blood as a sample for process testing.
2) Add D-PBS to the blood at a ratio of 1:2 and then invert the bottle to ensure that the blood and D-PBS are sufficiently mixed together, thus preparing a homogeneous blood sample.
3) Add 15 mL of Ficoll-Paque^{™} PLUS to a Leucosep tube, followed by centrifugation at 1,000 xg for 2 minutes. Carefully add 30 mL of the diluted blood into the prepared leucosep tube.
4) Centrifuge the leucosep tube prepared in 3) at 400 xg for 30 minutes at room temperature (15 to 25°C). *Change the centrifuge settings to acceleration-9 and deceleration-1.
5) Remove the plasma layer above the PBMCs.
6) Collect the PBMC layer in a fresh 50-mL tube. *Recover as much PBMCs as possible.
7) Add D-PBS to the recovered PBMCs at a 2:1 ratio. (e.g., 10 mL PBMC: 20 mL D-PBS).
8) Invert the tube prepared in 7), followed by centrifugation at 500 xg for 10 minutes at room temperature (15 to 25°C). Then, remove the supernatant.
9) Resuspend the cell pellet in each tube with 10 mL of D-PBS and collect the suspensions in one tube. Then, recover the cells remaining in the tube with 10 mL of D-PBS and perform this process twice more.
10) Count the cells resuspended in a total of 40 mL of D-PBS according to the "5.7.1 Live cell counting" method.
11) Centrifuge the remaining cells at 300 xg for 10 minutes.
12) Remove the supernatant, and then resuspend the cell pellet with 5% AB serum, 2 mM EDTA CliniMACS buffer (hereinafter "MACS buffer", VTRS03) to a concentration of 0.8 × 10⁷ cells/70 µL. * Carefully resuspend the cell pellet to become single cells.
13) Treat the cells with CliniMACS CD8 GMP MicroBeads at 0.8 × 10 ⁷ cells/30 µL.
14) Incubate the cells, treated with MACS buffer and beads, at room temperature (15 to 25°C) for 30 minutes.
15) Additionally add MACS buffer (VTRS03) to the incubated cells to a concentration of 1 × 10⁷ cells/mL and shake the cells, followed by centrifugation at 300 xg for 10 minutes at room temperature (15 to 25°C).
16) Prepare an LD column in QuadroMACS Separator.
17) Wet the column with 2 mL of MACS buffer (VTRS03). * Be careful not to dry the column.
18) Remove the supernatant after centrifugation in 15), and then resuspend the cells with MACS buffer (VTRS03) to a concentration of 1 × 10⁸ cells/500 µL.
19) Add the resuspended cells to the LD column.
20) Add 1 mL of MACS buffer (VTRS03) twice to the column and collect unlabeled cells in a 50 mL tube. * After confirming that the MACS buffer (VTRS03) completely flows down, it is added.
21) Add MACS buffer (VTRS03) to the cells collected in a 50 mL tube so that the final volume is 40 mL. Count the cells according to the "5.7.1 Live cell counting" method.
22) Centrifuge the remaining cells at 1,000 xg for 5 minutes at room temperature (15 to 25°C) .
23) Prepare a cell culture medium so that the number of cells counted is 4 × 10⁶ cells/mL, and then add IL-2 (500 unit/mL), bvPLA2 (0.4 pg/mL), and fibrillary amyloid-beta (5 µM) thereto according to the final volume.
24) Remove the supernatant from the centrifuged cells and resuspend the cells to a concentration of 8 × 10⁶ cells/mL.
25) Collect more than 5 × 10⁴ cells as a sample for process testing, based on the number of cells counted in 21) .
26) With reference to Table 1 below, dispense half of the final volume to be dispensed into a T75 Flask. Fill the flask with the cell culture medium, prepared in 23), to a concentration of 4 × 10⁶ cells/mL. After dispensing, dispense the remaining amount into an appropriate flask in the same manner, with reference to Table 1 below. However, the minimum culture volume of the T75 flask is set to 20 mL.
27) Mark the culture start date, preparation number, and person in charge on the surface of the flask, observe the cell state under a microscope, and then culture the cells in a 5% CO₂ incubator at 37°C for 3 days.

**[Table 1]**

| Cell concentration | | 1x10⁶ cells/mL |
|---|---|---|
| Concentration of ExpAct beads | | 1x10⁶ cells/40 µL |
| IL-2 concentration | | 1,000 unit/mL |

| Appropriate culture volume of flask | | |
|---|---|---|
| Flask type | Equal to or more than | Equal to or less than |
| T12.5 flask | 3 mL | < 5 mL |
| T25 flask | 5 mL | ≤ 7.5 mL |
| T75 flask | 15 mL | ≤ 22 mL |

However, when the cell number is less than 6 × 10⁶ cells, stand up the T12.5 flask and seed the cells in 3 mL. When the cell number is equal to or more than 6 × 10⁶ cells, seed the cells at 1 × 10⁶ cells/mL.

### 1-4. Isolation of Regulatory T Cells

Human regulatory T cells were isolated using CD4⁺CD25⁺CD127^{dim/-} Regulatory T Cell Isolation Kit II according to the following procedure.
1) Using a 240-mm scraper, scrape off cells attached to the bottom of a T75 flask.
2) Collect the cells, suspended in 1), in 50-mL tubes, and collect the cells remaining in the flask with 10 mL of MACS buffer (VTRS03), followed by centrifugation at 1,000×g for 5 minutes at 4°C.
3) After removing the supernatant, resuspend the cells with 10 mL of MACS buffer (VTRS03), and then collect the cells in one of four tubes. Then, collect the remaining cells in other tubes with 10 mL of MACS buffer (VTRS03).
4) Count the cells according to the "5.7.1 Live cell counting" method. Then, centrifuge the cells at 1,000 xg for 5 minutes at 4°C.
5) Remove the supernatant, and then resuspend the cells with MACS buffer (VTRS03) to a concentration of 0.8 × 10⁷ cells/40 µL.
6) Add CD4 ⁺CD25 ⁺CD127 ^{dim/-} T CELL Biotin-Antibody Cocktail II to the cells to a concentration of 0.8 × 10⁷ cells/10 µL.
7) Mix the treated cells well and incubate the cells at 4°C for 5 minutes.
8) To the incubated cells, add MACS buffer (VTRS03) to a concentration of 0.8 × 10⁷ cells/30 µL and anti-biotin MicroBeads to a concentration of 0.8 × 10⁷ cells/20 µL.
9) Mix the treated cells well and incubate the cells at 4°C for 10 minutes.
10) Prepare an LD column in QuadroMACS Separator.
11) Wet the LD column with 2 mL of MACS buffer (VTRS03). * Be careful not to dry the column.
12) Resuspend the cells, incubated in 9), with MACS buffer (VTRS03) to a concentration of 1×10⁸ cells/500 µL.
13) Add the resuspended cells to the LD column and 1 mL of MACS buffer (VTRS03) twice to the column. * After confirming that the MACS buffer (VTRS03) completely flows down, it is added.
14) Collect the cells, which fall down from the column, in a 15-mL tube, followed by centrifugation at 1,000 xg for 5 minutes at 4°C.
15) After removing the supernatant, resuspend the cells with MACS buffer (VTRS03) to a concentration of 0.8 × 10⁷ cells/90 µL.
16) Treat the resuspended cells with CD25 Micro beads II to a concentration of 0.8 × 10⁷ cells/10 µL, followed by mixing well and incubation at 4°C for 30 minutes.
17) Add 1 mL of MACS buffer (VTRS03) to the incubated cells, followed by centrifugation at 1,000 xg for 5 minutes at 4°C.
18) Prepare an MS column in an OctoMACS separator.
19) Wet the MS column with 1 mL of MACS buffer (VTRS03) .
20) Remove the supernatant after centrifugation in 17), and then resuspend the cells with MACS buffer (VTRS03) to a concentration of 1 × 10⁸ cells/500 µL.
21) Add the resuspended cells to the MS column, add 500 µL of MACS buffer (VTRS03) twice to the MS column, and collect the cells (CD25⁻ cells), which fall down from the column, in a 15 mL tube. * After confirming that the MACS buffer (VTRS03) completely flows down, it is added.
22) Add 1 mL of MACS buffer (VTRS03) to the MS column, apply pressure to the MS column by a plunger to detach the cells attached to the MS column, and collect the detached cells (CD25⁺ cells) in a tube. Perform this process once more (using a total of 2 mL of MACS buffer).
23) Centrifuge the 15-mL tube, prepared in 21), at 1,000 xg for 2 minutes at 4°C.
24) Prepare an MS column in an OctoMACS separator.
25) Wet the MS column with 1 mL of MACS buffer (VTRS03).
26) Remove the supernatant after centrifugation in 23), and then resuspend the cells with MACS buffer (VTRS03) to the same cell concentration as in 20).
27) Add the resuspended cells to the MS column, add 500 µL of MACS buffer (VTRS03) twice the column, and collect cells (CD25⁻ cells), which fall down from the column, in a 15 mL tube. * After confirming that MACS buffer (VTRS03) completely flows down, it is added.
28) Add 1 mL of MACS buffer (VTRS03) to the MS column, apply pressure to the MS column by a plunger to detach the cells attached to the MS column, and collect the detached cells (CD25⁺ cells) in a tube. Perform this process once more (using a total of 2 mL of MACS buffer).
29) After completion of the above procedure, a total of 4 mL of cells (CD25⁺ cells) attached to the MS column can be obtained.
30) Centrifuge the cells, recovered from the MS column, at 1,000×g for 5 minutes at 4°C.
31) Prepare an MS column in an OctoMACS separator.
32) Wet the MS column with 1 Ml of MACS buffer (VTRS03) .
33) Remove the supernatant after centrifugation in 30), and then resuspend the cells with MACS buffer (VTRS03) to the same cell concentration as in 20).
34) Add the cells, prepared in 33), to the MS column, add 500 µL of MACS buffer (VTRS03) twice to the column, and then cells (CD25⁻ cells), which fall down from the column, in a 15 mL tube.
35) Add 1 mL of MACS buffer (VTRS03) to the MS column, apply pressure to the MS column by a plunger to detach the cells attached to the MS column, and collect the detached cells (CD25⁺ cells) in a tube.
36) Perform the process of 35) once more (using a total of 2 mL of MACS buffer in an amount of 1 mL each time).
37) Centrifuge the cells, recovered from the MS column, at 1,000×g for 5 minutes at 4°C.
38) Resuspend the cells in 10 mL of a cell culture medium (VTRS04), and then count the cells, followed by centrifugation at 1,000 xg for 5 minutes at 4°C.

### 1-5. Passive Avoidance Test

Memory retention was measured using a fear-motivated avoidance system (Hamilton-Kinder LLC, San Diego, CA), and passive avoidance was observed by inhibition of previously expressed responses. The passive avoidance test, which consisted of a two-chamber box separated by a software-controlled mechanical gate, consisted of three training trials and one retention test. During the training trials, each mouse was placed on the right side of the chamber box with the lights turned off for 10 seconds for adaptation. After the 10-second adaptation period, the light in the right chamber was turned on, and then the guillotine door to the dark compartment on the left was opened. At this time, the mouse that entered the dark compartment received electrical stimulation. After completion of the electrical stimulation or after 180 seconds, the mouse was taken out of the box. In addition, the retention test was performed twice in the same manner as above, except that an electric shock was applied to the dark compartment. Latency and error times before the mouse entered the dark compartment were recorded, and it can be seen that, as the latency time increased and the error time decreased, the memory retention of the mouse for the dark compartment where the electrical stimulation had previously occurred was improved.

### 1-6. Y-Maze Test

The Y maze test was based on rodents' preference for exploring novel areas over familiar ones and was designed to measure several aspects of spatial working memory. The maze consists of three transparent plastic arms, 39 × 8 × 16 cm each, set at 120° angle relative to each other. During the first trial, mice could freely explore the start arm and the other arm for 5 min, and the third arm was blocked with an opaque door. Assignment of arms was counterbalanced randomly within each experimental group to avoid any preference-related bias. The mice were then returned to their home cage for 2 minutes. During the second trial, mice were placed at the extremity of their start arm and allowed to explore all three arms for 2 minutes. The maze was cleaned with 70% ethanol solution between the exploration phases to remove any olfactory cues.

### 1-7. RT-PCR

For RT-PCR, total RNA was extracted using the TRizol method and synthesized into complementary DNA using CycleScript Reverse Transcriptase (Bioneer, Daejeon, South Korea). PCR amplification was performed using a thermal cycler (Bio-Rad Lab., Hercules, USA). All reactions were performed using a Light-cycler 2.0 instrument (Roche Diagnostics, Mannheim, Germany) with SYBR Green I mix (Bioline, London, UK) according to the manufacturer's protocol. Primers used for amplification are shown in Table 2 below. PCR conditions for all primers were 95°C for 30 seconds, and 40 cycles, each consisting of 95°C for 15 seconds, and 60°C for 30 seconds. RT-qPCR data were calculated as the delta cycle threshold (ΔCT) values relative to β-actin, which is a housekeeping gene.

**[Table 2]**

| Gene | Sequence (5'→3') | |
|---|---|---|
| CD86 | Forward | GACCGTTGTGTGTGTTCTGG |
| | Reverse | GATGAGCAGCATCACAAGGA |
| NOS2 | Forward | CAGCTGGGCTGTACAAACCTT |
| | Reverse | CATTGGAAGTGAAGCGTTTCG |
| IL-12a | Forward | AAGCTCTGCATCCTGCTTCAC |
| | Reverse | GATAGCCCATCACCCTGTTGA |
| IL-1β | Forward | AAGCCTCGTGCTGTCGGACC |
| | Reverse | TGAGGCCCAAGGCCACAGG |
| IL-23 | Forward | CCTTCTCCGTTCCAAGATCCT |
| | Reverse | ACTAAGGGCTCAGTCAGAGTTGCT |
| IL-6 | Forward | TTCCATCCAGTTGCCTTCTTG |
| | Reverse | GGGAGTGGTATCCTCTGTGAAGTC |
| TNF-α | Forward | GGCAGGTTCTGTCCCTTTCAC |
| | Reverse | TTCTGTGCTCATGGTGTCTTTTCT |
| IL-4 | Forward | ATCCTGCTCTTCTTTCTCGAATGT |
| | Reverse | GCCGATGATCTCTCTCAAGTGATT |
| IL-10 | Forward | CAGCCGGGAAGACAATAACTG |
| | Reverse | CCGCAGCTCTAGGAGCATGT |
| TGF-β | Forward | GAGGTCACCCGCGTGCTA |
| | Reverse | TGTGTGAGATGTCTTTGGTTTTCTC |
| Arg1 | Forward | CTCCAAGCCAAAGTCCTTAGAG |
| | Reverse | AGGAGCTGTCATTAGGGACATC |
| Ym1 | Forward | TGGAGGATGGAAGTTTGGAC |
| | Reverse | GAGTAGCAGCCTTGGAATGT |
| Mrc1 | Forward | TTCGGTGGACTGTGGACGAGC |
| | Reverse | ATAAGCCACCTGCCACTCCGG |
| IL-21 | Forward | TGCTAGCTCCAGCCTCAGTCT |
| | Reverse | TTAAGTGCTGAACTTGTTTGGATTG |
| Trem2 | Forward | CTACCAGTGTCAGAGTCTCCGA |
| | Reverse | CCTCGAAACTCGATGACTCCTC |
| Tmem119 | Forward | GGATAGTGGACTTCTTCCGCCA |
| | Reverse | GGAAGGACGATGGGTAATAGGC |
| Cx3cr1 | Forward | GAGCATCACTGACATCTACCTCC |
| | Reverse | AGAAGGCAGTCGTGAGCTTGCA |
| Itgax | Forward | TGCCAGGATGACCTTAGTGTCG |
| | Reverse | CAGAGTGACTGTGGTTCCGTAG |
| Ccl6 | Forward | CACCAGTGGTGGGTGCATCAAG |
| | Reverse | GTGCTTAGGCACCTCTGAACTC |
| Csf1 | Forward | GCCTCCTGTTCTACAAGTGGAAG |
| | Reverse | ACTGGCAGTTCCACCTGTCTGT |
| β-Actin | Forward | GTGCTATGTTGCTCTAGACTTCG |
| | Reverse | ATGCCACAGGATTCCATACC |

**1-8. Western Blotting** Cells were lysed with proprep (iNtRON Biotechnology, Seongnam, South Korea) at 4°C for 20 minutes. The protein concentration of each sample was determined by Bradford assay. Each sample (30 µg of protein) was resolved on an 8% SDS-polyacrylamide gel, transferred to a nitrocellulose membrane, and blocked in 5% skimmed milk at room temperature (20°C to 25°C) for 30 minutes. After 30 minutes of blocking, the membrane was reacted sequentially with primary antibodies (anti-CD86, anti-NOS2 or anti-β-actin) for overnight incubation at 4°C. After washing with Tris-buffered saline with Tween 20 (TBS-T) containing 25 mM Tris-Cl, 150 mM NaCl, and 0.05% Tween-20, blots were incubated with HRP-conjugated secondary antibodies (anti-rabbit or anti-mouse IgG) for 2 hours. Immunoreactivity was visualized using a Western Blotting Detection Reagent Kit (Abclon, Seoul, Korea). Antibody binding was determined using a chemiluminescent detection system (Davinch-Chemi, Seoul, South Korea). Densitometric analysis was performed using Image J software.

### 1-9. Immunohistochemical staining (IHC)

The mice were anesthetized with isoflurane, and then perfused transcardially with ice-cold PBS followed by a solution of 4% paraformaldehyde in PBS. The brain was harvested, fixed in 4% paraformaldehyde solution at 4°C for 48 hours, and then stored in 30% sucrose/PBS solution at 4°C. After bisecting the brain along the midline, each hem-brain was divided into anterior, median and posterior parts by coronal sectioning. By coronal sectioning. Each part was then embedded individually in Optimal Cutting Temperature (OCT; WVR) mounting medium, frozen in liquid nitrogen, and stored at -80°C. Cryosections (7 um) were prepared from median parts of the brain, using a Leica RM2145 cryostat, and mounted on SuperFrost^{™} Plus glass slides. At least three nonadjacent sections (100 µm apart) were labeled for each mouse. Each section was first rinsed with PBS, blocked with PBS-5% BSA-0.05% Tween 20 for 1 hour at room temperature, and then incubated either overnight at 4°C with rabbit anti-Iba1 antibody (1/200) (Wako) or for 30 minutes at room temperature with rabbit anti-GFAP antibody (1/200) (Dako). After washing three times with PBS-0.1% BSA, the sections were incubated for 30 min at room temperature with anti-CD11b (1/200) (ab8878; Abcam), washed 3 times, and incubated for 30 min at room temperature, and then incubated with Alexa Fluor^{®} 488-conjugated goat anti-rat and Alexa Fluor^{®} 594-conjugated goat anti-goat antibodies (1/1,000 each) at room temperature for 30 minutes. After washing with 3 times, the brain sections were stained with DAPI (1 µg/ml in PBS) and coverslipped with Immu-Mount medium (ThermoShandon). Images were obtained using Zen2 (blue edition) software and a confocal fluorescence microscope (LSM800, Carl Zeiss). Quantitative analysis of microglia in all fields/images was performed based on pixel intensity, and cells were counted using Image J (National Institutes of Health).

### 1-10. Statistical Analysis

Statistical analysis was performed using GraphPad Prism software (GraphPad, La Jolla, CA, United States). Conditions were compared using one-way ANOVA analysis followed by post-hoc group comparison. Data were expressed as mean ± SEM, and P < 0.05 was considered significant.

### <Example 2> Experimental Results

### 2-1. Therapeutic Effect of Tregs in Alzheimer's Disease

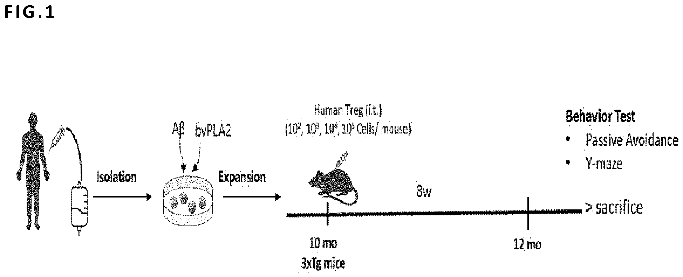

To investigate the therapeutic effect of Tregs in Alzheimer's disease, human regulatory T cells (hTregs) were isolated and expanded *ex vivo* (FIG. 1). The expanded hTregs were administered intrathecally to 3xTg AD mice at cell doses of 1×10² cells/mouse, 1×10³ cells/mouse, 1×10⁴ cells/mouse and 1×10⁵ cells/mouse. Control mice were administered PBS only using the same administration method.

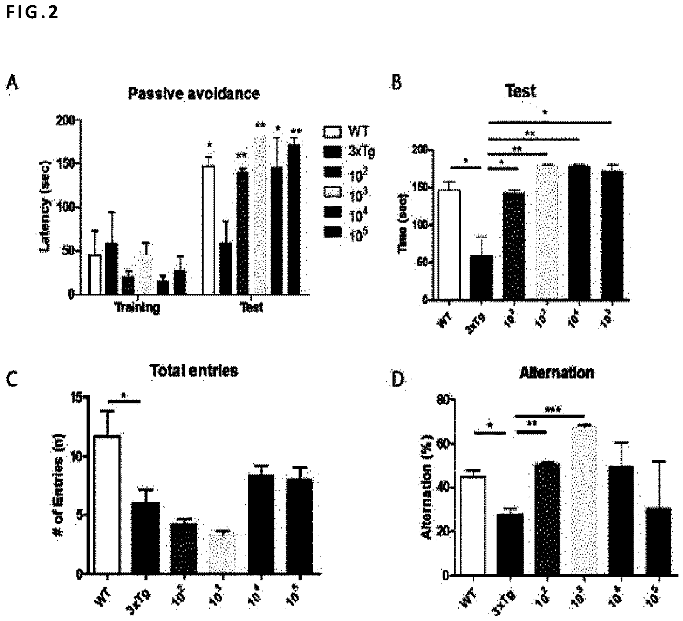

As a result of the passive avoidance test, the 3xTg AD mice did not show improvement in latency time (memory) (FIG. 2A). In contrast, the difference in mean retention latency time between WT mice and the 3xTg AD mice reached statistical significance (FIG. 2B). Training latency also showed significant differences in all of the hTreg-administered groups (10², 103, 10⁴, and 10⁵) (p = 0.0361, 0.0091, 0.0096, and 0.0143, respectively).

Then, 10-month-old mice were evaluated for short-term attention memory. As a result of the Y-maze test, all items significantly decreased only in the 3xTg AD mouse group compared to the WT group (FIG. 2C), and spontaneous alteration significantly changed in the 3xTg AD mouse group compared to the WT mice, and also significantly changed in the 10² and 10³ hTreg-administered groups compared to the WT group (FIG. 2D). These results indicate that administration of hTregs significantly improved the learning and memory abilities of the Alzheimer's disease mice.

### 2-2. Effect of hTregs on M1 microglia and disease-associated microglia (DAM)

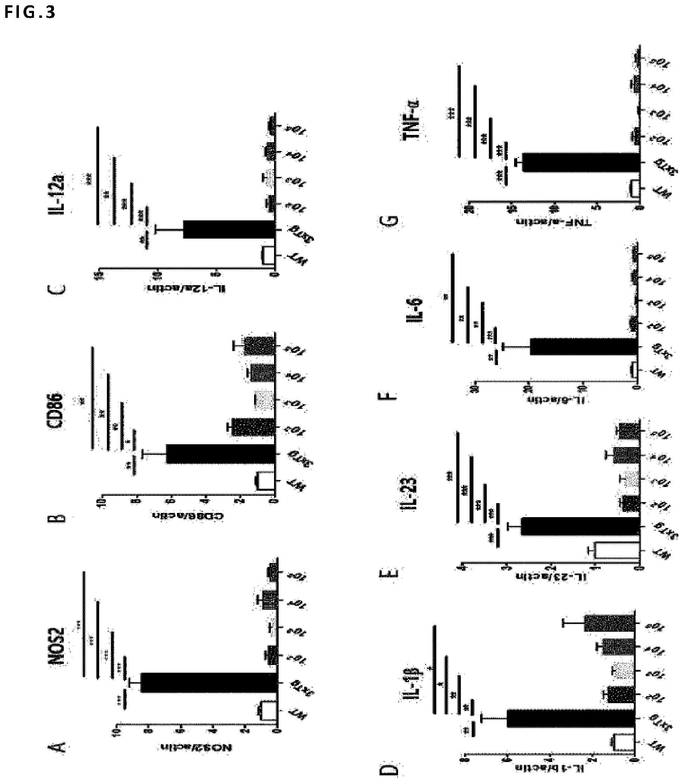

First, real-time qPCR was performed to evaluate the direct effect of hTregs on the mRNA expression of M1 microglia markers in the hippocampus. As a result, it was confirmed that the expression of NOS2, CD86, IL-12a, IL-1β, IL-1β, IL-23, IL-6, and TNF-α increased in the 3xTg AD mice, but the expression of these markers in the hTreg-administered mice the expression was similar to that in the WT mice (FIG. 3).

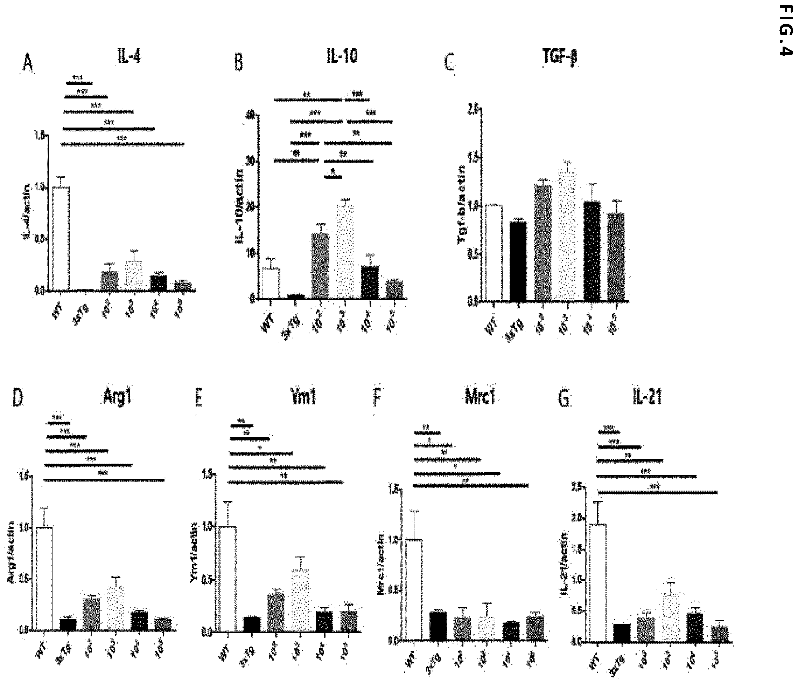

Real-time PCR was performed to investigate the mRNA expression of disease-associated microglia (DAM) markers in the hippocampus. Similar to the results in Example 2-1, the expression of DAM markers was found to increase in the 3xTg AD mice. However, hTregs significantly suppressed the expression of Trem2, Tmem119, Cc3cr1, Itgax, and Ccl6 (FIG. 4) .

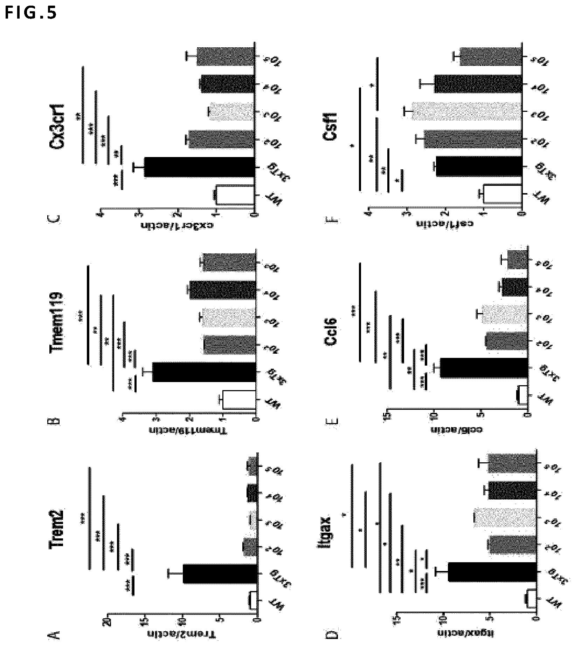

Subsequently, the present inventors also measured the protein expression of M1 (FIG. 5A), M2 (FIG. 5B) microglia and nerve growth factor (FIG. 5C) and DAM (FIG. 5D) by Western blot. The expression level of CD86 in the 3xTg AD group increased compared to that in the WT group (p < 0.01). The protein expression level of CD86 in the hippocampus decreased in all of the hTreg-treated groups compared to the 3xTg AD group (FIG. 5A, p < 0.01). Western blot analysis of the expression of NOS2, Arg1, CD206, Iba-1 and BDNF showed no significant changes in protein levels (FIGS. 5B and 5C). However, the expression of Trem2 expression significantly decreased in the 10⁵ hTreg-injected group compared to the 3xTg AD group (FIG. 5D).

These results indicate that M1 microglia are activated in 3xTg AD mice, and hTreg suppresses the expression of M1 microglia and DAM markers that increased by Alzheimer's disease, thereby suppressing M1 microglial activation and transformation into DAM.

### 2-3. Effect of hTregs on mRNA expression of M2 microglia markers

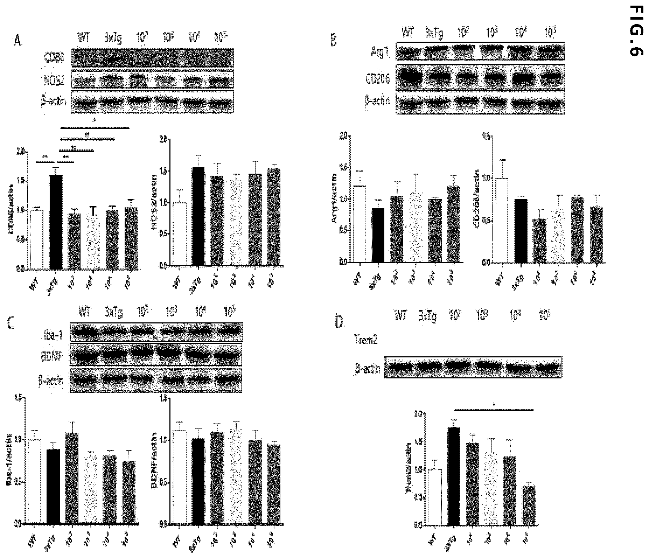

Real-time qPCR was performed to evaluate the direct effect of hTregs on the mRNA expression of M2 microglia markers in the hippocampus. As a result, in contrast to the results in Example 2-1, the expression of M2 microglia-related markers IL-4, IL-10, TGF-β, Arg1, Ym1, Mrc1, and IL-2 significantly decreased in the 3xTg AD mice. In addition, the expression of IL-10 was found to significantly increase in the mice administered 10² and 10³ hTregs (FIG. 6).

### 2-4. Analysis of Immunoreactivity for CD11b and Trem2

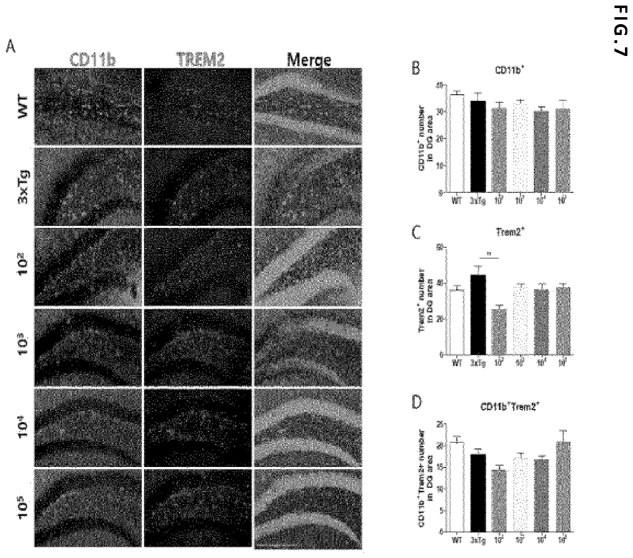

Immunoreactivity for CD11b and Trem2 was observed in the hippocampal dentate gyrus (DG) (FIG. 7A). In particular, Trem2+ cells significantly decreased in the 10² hTreg-administered group compared to the 3xTg AD group, but did not decrease in other experimental groups (FIG. 7C). No significant changes between CD116b+ cells and CD116b+Trem2+ cells were observed in all of the experimental groups (FIGS. 7B and 7D). These results indicate that hTregs selectively inhibited Trem2 without changing CD11b.

Trem2 is a receptor expressed on the surface of microglia, which are immune cells in the brain, and it plays a role in allowing microglia to transform into disease-associated microglia (DAM). Therefore, these results suggest that hTregs have the effect of suppressing the transformation of microglia into disease-associated microglia (DAM).

### 2-5. Effect of Tregs against Amyloid-Beta and Tau Proteins

To understand how Tregs affect the progression of Alzheimer's disease, the present inventors investigated whether intrathecal administration of Tregs could reduce phosphorylation of tau protein in the hippocampus of an AD mouse model.

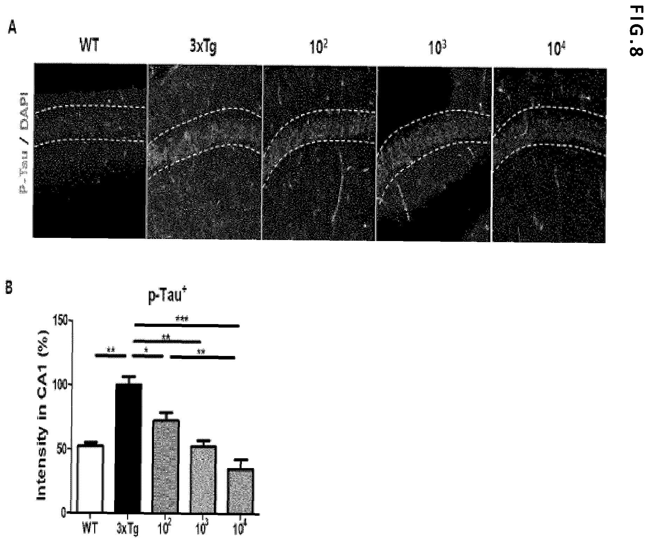

Specifically, to examine the change in the expression level of p-Tau depending on the number of hTreg cells administered, 10-month-old 3xTg AD mice were injected with 1 × 10², 1 × 103, or 1 × 10⁴ hTregs/mouse or PBS. Then, immunofluorescence staining for phosphorylated tau protein (p-Tau) in the hippocampus of the 12-month-old mice was performed, and the results are shown in FIGS. 8A and 8B.

As shown in FIGS. 8A and 8B, it was found that the level of phosphorylated tau protein in the hippocampus of the control 3xTg AD mice significantly increased, and the level of phosphorylated tau protein significantly decreased in the hTreg-treated group compared to the 3xTg AD mice not treated with hTregs (FIGS. 8A and 8B). In particular, it was confirmed that the expression level of p-Tau significantly decreased even in the mice administered 1 × 10² hTregs, which is a very small amount.

Taking these results together, hTregs suppress M1 activation of microglia, the innate immune cells of the central nervous system (CNS) that maintain homeostasis of inflammatory processes and regulate cognitive function, and promote M2 polarization of microglia, thus removing tau protein and inducing neuroprotective effects. Therefore, these results indicate that human regulatory T cells (hTregs) may be used as a composition for preventing or treating central nervous system diseases, especially degenerative brain diseases.

## Claims

1. A pharmaceutical composition for preventing or treating degenerative brain disease comprising regulatory T cells as an active ingredient.

2. The pharmaceutical composition of claim 1, wherein the regulatory T cells are human regulatory T cells.

3. The pharmaceutical composition of claim 1, wherein the regulatory T cells inhibit expression of NOS2, CD86, IL-12a, IL-1β, IL-1β, IL-23, IL-6, TNF-α, Trem2, Tmem119, Cc3cr1, Itgax, and Ccl6.

4. The pharmaceutical composition of claim 1, wherein the regulatory T cells increase expression of IL-4, IL-10, TGF-β, Arg1, Ym1, Mrc1, and IL-2.

5. The pharmaceutical composition of claim 1, wherein the degenerative brain disease is any one selected from the group consisting of Alzheimer's disease, Parkinson's disease, dementia, progressive supranuclear palsy, multiple system atrophy, olivopontocerebellar atrophy (OPCA), Shy-Drager syndrome, striatonigral degeneration, Huntington's disease, amyotrophic lateral sclerosis (ALS), essential tremor, corticobasal ganglionic degeneration, diffuse Lewy body disease, Parkinson-ALS-dementia complex of Guam, and Pick's disease.

6. The pharmaceutical composition of claim 1, wherein the composition is for intrathecal administration.

7. A method for preventing or treating degenerative brain disease, comprising a step of administering the pharmaceutical composition of claim 1 to a subject.

8. The method of claim 7, wherein the regulatory T cells are human regulatory T cells.

9. The method of claim 7, wherein the pharmaceutical composition is for intrathecal administration.
